# EUROPÄISCHE PATENTANMELDUNG

(11) **EP 1 764 880 A2**
(43) Veröffentlichungstag der Anmeldung: **21.03.2007**
(21) Anmeldenummer: 06018488.4
(22) Anmeldetag: 02.09.2006
(51) Int. Cl.: H01R 13/66

(54) **Elektrisches Installationsgerät zur Duftverteilung**

(30) Priorität: 17.09.2005 DE 102005044541
(71) Anmelder: ABB PATENT GmbH, 68526 Ladenburg (DE)
(72) Erfinder: Dörstel, Bernhard, Dipl.-Ing., 50321 Brühl (DE)
(74) Vertreter: Miller, Toivo

(57) **Zusammenfassung**

Es wird ein elektrisches Installationsgerät zur Duftverteilung mit einem in eine Standard-Unterputzdose einbaubaren Gerätesockel (1) vorgeschlagen, welcher einen Ventilator (12, 23, 25) oder eine elektrisch betätigbare Luftklappe (30) enthält sowie mit einem frontseitigen Tragring (2) und zwei sich gegenüberliegenden seitlichen Befestigungsspreizen (3) versehen ist und mit einem am Tragring (2) befestigbaren Abdeckrahmen (5) inklusive einer Duftstoff-Aufnahmekammer (7), welche mittels einer mit Öffnungen (11) für den Luftaustritt und gegebenenfalls Lufteintritt versehenen Abdeckung (10) verschlossen ist.

## Beschreibung

Die Erfindung bezieht sich auf ein elektrisches Installationsgerät zur Duftverteilung.

Aus der DE 298 17 599 U1 ist eine elektrische Ventilatoranordnung bekannt, die zum Verströmen einer wohlduftenden Essenz oder Parfum zu vorbestimmten Zeitintervallen betrieben werden kann. Dabei ist an einer elektrischen Ventilatoreinheit ein Gefäß angebracht, welches ein aromatisches Präparat enthält. Dabei handelt es sich um ein tragbares Einzelgerät, welches an gewünschter Stelle innerhalb eines Raumes aufzustellen oder aufzuhängen ist.

Aus der DE 295 14 353 U1 ist ein Duftspender bekannt, welcher einen Ventilator aufweist, dessen erzeugter Luftstrom einen aus einem Vorratsbehälter mit Duftstoff versorgten Verdunstungskörper umströmt, wobei der Duftspender von einem geschlossenen Gehäuse umgeben ist, an welchem direkt ein Netzstecker zur Stromversorgung des Ventilators angeordnet ist.

Der Erfindung liegt die Aufgabe zugrunde, ein elektrisches Installationsgerät zur Duftverteilung anzugeben, welches keinerlei Platzbedarf zum Aufstellen im Raum erfordert, wie dies z. B. bei einem Einzelgerät der Fall ist.

Diese Aufgabe wird erfindungsgemäß gelöst durch ein elektrisches Installationsgerät zur Duftverteilung mit einem in eine Standard-Unterputzdose einbaubaren Gerätesockel, welcher einen Ventilator oder eine elektrisch betätigbare Luftklappe enthält sowie mit einem frontseitigen Tragring und zwei sich gegenüberliegenden seitlichen Befestigungsspreizen versehen ist und mit einem am Tragring befestigbaren Abdeckrahmen inklusive einer Duftstoff-Aufnahmekammer, welche mittels einer mit Öffnungen für den Luftaustritt und gegebenenfalls Lufteintritt versehenen Abdeckung verschlossen ist.

Die mit der Erfindung erzielbaren Vorteile bestehen insbesondere darin, dass das Installationsgerät zur Duftverteilung vom Design her in ein Installationsprogramm integriert werden kann und damit in die Elektroinstallation eingebunden wird. Für unterschiedliche Unterputzprogramme werden jeweils passende Abdeckrahmen inklusive Duftstoff-Aufnahmekammern und Abdeckungen eingesetzt. Dabei ist wahlweise eine Betätigung über ein manuelles Schaltgerät oder über ein Hausbussystem möglich. Insgesamt handelt es sich um einen sehr unauffälligen Duftspender, d. h. auf den ersten Blick ist die "Quelle" der Dufterzeugung überhaupt nicht erkennbar.

Weitere Vorteile sind aus der nachstehenden Beschreibung ersichtlich.

Vorteilhafte Ausgestaltungen der Erfindung sind in den Unteransprüchen gekennzeichnet.

Die Erfindung wird nachstehend anhand der in der Zeichnung dargestellten Ausführungsbeispiele erläutert. Es zeigen:
- Fig.1: ein elektrisches Installationsgerät zur Duftverteilung in "Explosionsdarstellung",
- Fig. 2: die elektrische Verschaltung eines elektrisches Installationsgeräts zur Duftverteilung,
- Fig. 3: eine Ansteuerung eines elektrisches Installationsgeräts zur Duftverteilung über einen Bus eines Gebäudeinstallationssystems,

- Fig. 4 - 8: unterschiedliche Ausführungsformen eines elektrischen Installationsgeräts zur Duftverteilung,
- Fig. 9: eine alternative Ausführungsform eines elektrisches Installationsgerätes zur Duftverteilung.

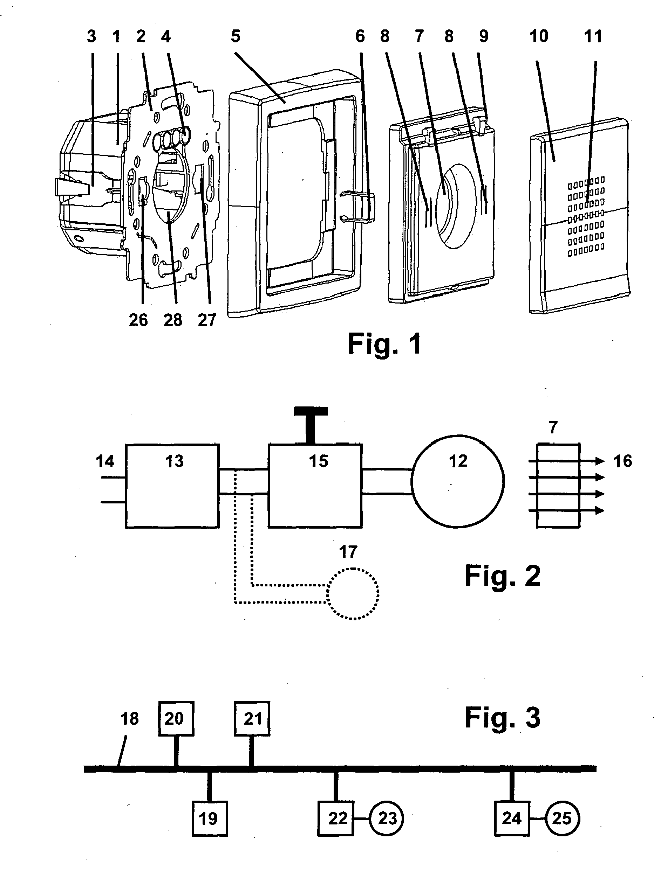

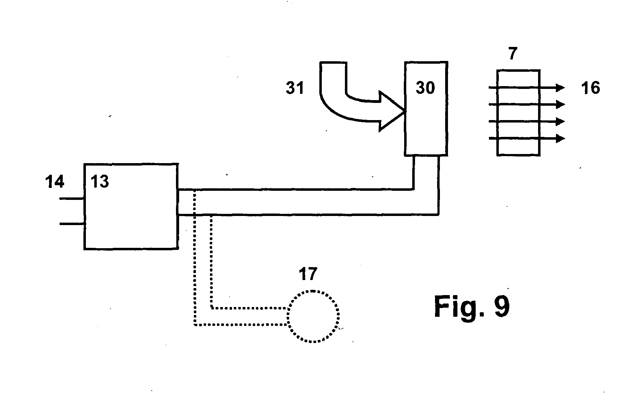

In Fig. 1 ist ein elektrisches Installationsgerät zur Duftverteilung in "Explosionsdarstellung" dargestellt. Das elektrische Installationsgerät besteht aus einem einen Ventilator (siehe Ziffer 12 in Fig. 2) oder eine elektrisch betätigbare Luftklappe (siehe Ziffer 30 in Fig. 9) aufweisenden Gerätesockel 1 mit frontseitigem Tragring 2 sowie Befestigungsspreizen 3 an zwei sich gegenüberliegenden Seitenflächen, so dass der Einbau des Installationsgerätes in eine Standard-Unterputzdose in einfacher Weise möglich ist. Die Verbindungen mit in den Innenraum der Unterputzdose eingeführten elektrischen Leitungen erfolgt über Anschlussklemmen 4. Der Tragring 2 weist zwei seitliche Luft-Einströmöffnungen 26, 27 und eine zentrale Luft-Ausströmöffnung 28 auf. Nach erfolgter Montage in einer Standard-Unterputzdose wird das elektrische Installationsgerät komplettiert
- mit einem Abdeckrahmen 5 mit gewünschtem Design, welcher z. B. unter Zuhilfenahme von Rastmitteln 6 am Tragring 2 befestigbar ist,
- mit einer Duftstoff-Aufnahmekammer 7, welche in den Abdeckrahmen 5 einsetzbar ist und eine zentrale Öffnung zur Einlage eines Duftpads sowie seitlich angeordnete Luftschlitze 8 zum Eintritt von Raumluft aufweist,
- mit einer Abdeckung 10, welche Öffnungen 11 zum Eintritt von Raumluft sowie zum Austritt von mit Duftstoff angereicherter Luft aufweist und vorzugsweise in Form eines Klappdeckels ausgebildet ist, welcher über Scharniere an Gelenkzapfen 9 der Duftstoff-Aufnahmekammer 8 angelenkt ist.

Zum Austausch eines verbrauchten Duftpads gegen ein neues Duftpad wird die Abdeckung 10 entfernt bzw. eine als Klappdeckel ausgebildete Abdeckung 10 geöffnet, worauf der Duftpad-Wechsel in einfacher Weise möglich ist.

Die Abdeckung 10 kann in unterschiedlicher Weise ausgebildet sein, um z. B. eine variable Ausströmrichtung zu realisieren:
- beispielsweise mit Öffnungen 11, welche eine Luftströmung in eine bestimmte Austrittsrichtung erzeugen, beispielsweise nach unten, nach oben oder in eine gewünschte seitliche Richtung,
- beispielsweise mit verstellbaren Lamellen, durch die eine Lüftströmung in eine gewünschte Austrittsrichtung erzeugt werden kann.

Selbstverständlich können in einer einfacheren Ausführung Abdeckrahmen 5 und Duftstoff-Aufnahmekammer 7 als einteilige Komponente ausgebildet sein.

In Fig. 2 ist die elektrische Verschaltung eines einen Ventilator aufweisenden elektrischen Installationsgeräts zur Duftverteilung dargestellt. Der vorstehend bereits erwähnte Ventilator 12 ist vorzugsweise über eine Drehzahleinstellvorrichtung 15 mit einem Schaltelement 13 verbunden, welches an einem Netz 14 liegt. Bei einfachster Ausführung kann ein Potentiometer als Drehzahleinstellvorrichtung 15 eingesetzt werden. Dabei kann die Drehzahleinstellvorrichtung 15 wahlweise
- im elektrischen Installationsgerät zur Duftverteilung selbst enthalten sein,
- als separates elektrisches Installationsgerät für Unterputz-Einbau ausgebildet sein.
   Nach Einschalten des Ventilators 12 wird eine Luftströmung 16 durch die Duftstoff-Aufnahmekammer 7 erzeugt, wobei die Luft durch die Öffnungen 11 sowie die Luft-Einströmöffnungen 26, 27 seitlich eintritt und mit Duftstoff angereichert zentral durch die Luft-Ausströmöffnung 28 und die Öffnungen 11 austritt.

Beim Schaltelement 13 kann es sich wahlweise handeln:
- um ein im Gerätesockel des elektrischen Installationsgerätes integriertes Schaltelement,
- um ein separates elektrisches Installationsgerät (Schaltgerät) für Unterputz-Einbau,
- um einen Lichtschalter, welcher eine Leuchte 17 eines Raumes schaltet, wobei gleichzeitig mit dieser Leuchte 17 auch der Ventilator 12 des Installationsgerätes zur Duftverteilung eingeschaltet/ausgeschaltet wird,
- um einen Schaltkontakt eines separaten Bewegungsmelders,
- um einen Schaltkontakt eines im Installationsgerät zur Duftverteilung integrierten Bewegungsmelders.
   Bei den Versionen mit Bewegungsmelder wird der Ventilator 12 des Installationsgerätes zur Duftverteilung mittels dieses Bewegungsmelders bei Anwesenheit einer detektierten Person eingeschaltet und ausgeschaltet, sobald die Person den detektierten Bereich wieder verlässt.

In Fig. 3 ist eine Ansteuerung eines elektrischen Installationsgeräts zur Duftverteilung über einen Bus (Kommunikationssystem) eines Gebäudeinstallationssystems dargestellt. Im Ausführungsbeispiel ist ein Bus (Kommunikationssystem) 18 eines Gebäudeinstallationssystems zu erkennen, an welches u. a. folgende Baukomponenten angeschlossen sind:
- ein zentrales Steuergerät 19,
- ein Schaltsensor 20,
- ein Schaltsensor 21,
- ein Aktor 22, welcher einen Ventilator 23 eines Installationsgerätes zur Duftverteilung ansteuert,
- ein Aktor 24, welcher einen Ventilator 25 eines Installationsgerätes zur Duftverteilung ansteuert.

Durch den Schaltsensor 20 wird der Aktor 22 angesteuert. Durch den Schaltsensor 21 wird der weitere Aktor 24 angesteuert. Die Aktoren 22, 24 (elektronische Steuereinheiten) können wahlweise im Gerätesockel 1 des Installationsgerätes zur Duftverteilung integriert oder als separate Installationsgeräte mit eigenem Gerätesockel ausgebildet sein. Die nach Ansteuerung der Ventilatoren 23, 25 verströmten Duftstoffe sind vorzugsweise unterschiedlicher Art, so dass die Möglichkeit gegeben ist, die Raumluft zu verschiedenen Zeiten mit unterschiedlichen Duftstoffen anzureichern. Wie allgemein bei einem Bus der Gebäudeinstallation bekannt, erfolgt über das zentrale Steuergerät 19 jeweils eine Umsetzung der durch die Schaltsensoren bzw. allgemein Sensoren vorgegebenen Befehle in entsprechende über Aktoren auszuführende Handlungen. Selbstverständlich können weitere Aktoren mit jeweils nachgeschalteten Ventilatoren vorgesehen sein. Auf diese Art und Weise ist ein "Wellness-Szenario" realisierbar, bei welchem eine Vielzahl von unterschiedlichen Duftstoffen in gewünschter zeitlicher Abfolge produzierbar und einem Raum zuführbar ist.

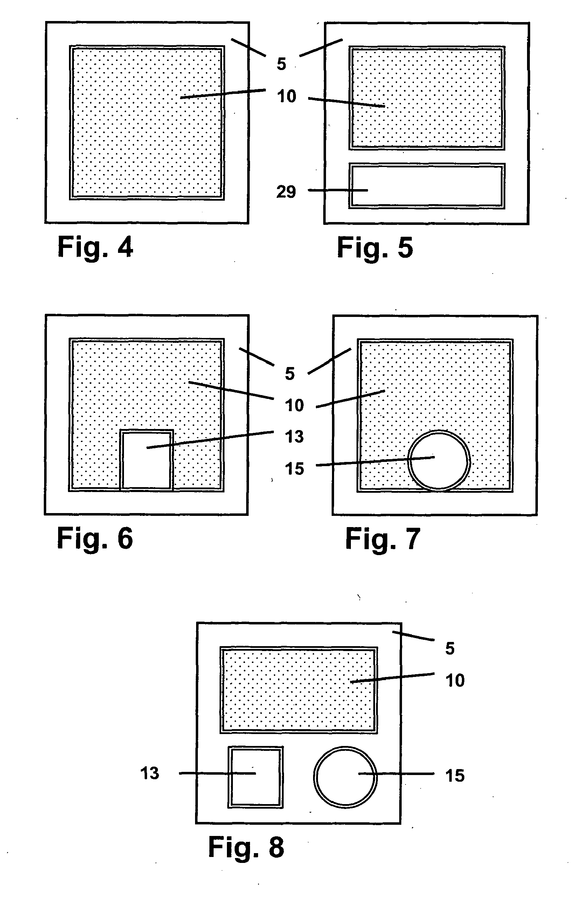

In den Fig. 4 - 8 sind die Ansichten auf die Frontseiten von unterschiedlichen Ausführungsformen eines elektrisches Installationsgeräts zur Duftverteilung dargestellt:
- Fig. 4 zeigt die Grund-Ausführung mit Abdeckrahmen 5 und Abdeckung 10.
- Fig. 5 zeigt eine Ausführungsform mit Abdeckrahmen 5, Abdeckung 10 und integriertem Bewegungsmelder 29.
- Fig. 6 zeigt eine Ausführungsform mit Abdeckrahmen 5, Abdeckung 10 und integriertem Schaltelement 13.
- Fig. 7 zeigt eine Ausführungsform mit Abdeckrahmen 5, Abdeckung 10 und integrierter Drehzahleinstellvorrichtung 15.
- Fig. 8 zeigt eine Ausführungsform mit Abdeckrahmen 5, Abdeckung 10 und integriertem Schaltelement 13 / integrierter Drehzahleinstellvorrichtung 15.
   In Fig. 9 ist eine alternative Ausführungsform eines elektrischen Installationsgerätes zur Duftverteilung dargestellt. Bei dieser Ausführungsform wird eine elektrisch betätigbare Luftklappe 30 an Stelle eines Ventilators eingesetzt. Das elektrische Installationsgerät zur Duftverteilung ist über eine Luftzuführung 31 an ein im Gebäude verlegtes Luft-System (z. B. von einer Raumbelüftung abgeleitet) angeschlossen. In geöffneter Stellung der Luftklappe 30 wird die vorstehend erwähnte mit Duftstoff angereicherte Luftströmung 16 erzeugt. Die Ansteuerung der Luftklappe 30 kann über ein Schaltelement 13 erfolgen, das auch eine Leuchte 17 des Raumes schaltet, wie vorstehend erwähnt. Die Ansteuerung der Luftklappe kann selbstverständlich auch über einen Aktor und einen Bus eines Gebäudeinstallationssystem erfolgen, wie dies vorstehend für die Ausführungsform mit Ventilator beschrieben ist.

### Bezugszeichenliste:

- 1: Gerätesockel
- 2: Tragring
- 3: Befestigungsspreizen
- 4: Anschlussklemmen
- 5: Abdeckrahmen
- 6: Rastmittel
- 7: Duftstoff-Aufnahmekammer
- 8: Luftschlitze
- 9: Gelenkzapfen
- 10: Abdeckung, vorzugsweise in Form eines Klappdeckels
- 11: Öffnungen
- 12: Ventilator
- 13: Schaltelement
- 14: Netz
- 15: Drehzahleinstellvorrichtung
- 16: Luftströmung
- 17: Leuchte
- 18: Bus (Kommunikationssystem) eines Gebäudeinstallationssystems
- 19: zentrales Steuergerät
- 20: Schaltsensor
- 21: Schaltsensor
- 22: Aktor
- 23: Ventilator
- 24: Aktor
- 25: Ventilator
- 26: Luft-Einströmöffnung
- 27: Luft-Einströmöffnung
- 28: Luft-Ausströmöffnung
- 29: Bewegungsmelder
- 30: elektrisch betätigbare Luftklappe
- 31: Luftzuführung

## Patentansprüche

1. Elektrisches Installationsgerät zur Duftverteilung mit einem in eine Standard-Unterputzdose einbaubaren Gerätesockel (1), welcher einen Ventilator (12, 23, 25) oder eine elektrisch betätigbare Luftklappe (30) enthält sowie mit einem frontseitigen Tragring (2) und zwei sich gegenüberliegenden seitlichen Befestigungsspreizen (3) versehen ist und mit einem am Tragring (2) befestigbaren Abdeckrahmen (5) inklusive einer Duftstoff-Aufnahmekammer (7), welche mittels einer mit Öffnungen (11) für den Luftaustritt und gegebenenfalls Lufteintritt versehenen Abdeckung (10) verschlossen ist.

2. Elektrisches Installationsgerät nach Anspruch 1, **dadurch gekennzeichnet, dass** die Abdeckung (10) in Form eines Klappdeckels ausgebildet ist.

3. Elektrisches Installationsgerät nach Anspruch 2, **dadurch gekennzeichnet, dass** die Abdeckung (10) gerichtete Öffnungen (11) oder Lamellen aufweist, wodurch eine variable Ausströmrichtung realisierbar ist.

4. Elektrisches Installationsgerät nach einem der vorstehenden Ansprüche, **dadurch gekennzeichnet, dass** Abdeckrahmen (5) und Duftstoff-Aufnahmekammer (7) als getrennte Baukomponenten ausgebildet sind.

5. Elektrisches Installationsgerät nach einem der vorstehenden Ansprüche, **dadurch gekennzeichnet, dass** der Gerätesockel (1) ein den Ventilator (12) oder die Luftklappe (30) schaltendes Schaltelement (13) enthält.

6. Elektrisches Installationsgerät nach einem der vorstehenden Ansprüche, **dadurch gekennzeichnet, dass** der Gerätesockel (1) eine den Ventilator (12) beeinflussende Drehzahleinstellvorrichtung (15) enthält.

7. Elektrisches Installationsgerät nach einem der vorstehenden Ansprüche, **dadurch gekennzeichnet, dass** der Gerätesockel (1) einen den Ventilator (12) oder die Luftklappe (30) ansteuernden Bewegungsmelder (29) enthält.

8. Elektrisches Installationsgerät nach einem der vorstehenden Ansprüche, **dadurch gekennzeichnet, dass** der Gerätesockel (1) einen zum Anschluss an einen Bus (18) eines Gebäudeinstallationssystems geeigneten Aktor (22, 24) enthält.
